# EUROPÄISCHE PATENTANMELDUNG

(11) **EP 1 250 902 A1**
(43) Veröffentlichungstag der Anmeldung: **23.10.2002**
(21) Anmeldenummer: 01108892.9
(22) Anmeldetag: 10.04.2001
(51) Int. Cl.: A61F 15/00, A61J 1/00, B65D 81/32, A61K 7/48, A45D 44/00

(54) **Pflegesystem zur kosmetischen Behandlung der Haut**

(71) Anmelder: Böttger GmbH Pharmazeutische und Kosmetische Präparate, 10709 Berlin (DE)
(72) Erfinder: Beyer, Ursula Dipl. Ing., 14558 Bergholz-Rehbrücke (DE)
(74) Vertreter: Stenger, Watzke & Ring Patentanwälte

(57) **Zusammenfassung**

Die Erfindung betrifft ein Pflegesystem zur kosmetischen Behandlung der Haut durch Auflage eines pflegestoffhaltigen Flächenelements auf die zu behandelnden Hautpartien, umfassend wenigstens ein Textilerzeugnis, welches im wesentlichen flüssigkeitsfrei zusammengepreßt ist, sowie eine Flüssigkeit, wobei das Textilerzeugnis und/oder die Flüssigkeit Pflegestoffe aufweisen und das Textilerzeugnis nach Aufnahme der Flüssigkeitzu einem pflegestoffhaltigen Flächenelement entfaltbar ist. Das erfindungsgemäße Pflegesystem zeichnet sich durch eine einfache und hygienische Anwendung aus, sowie dadurch, daß es wenig zeitaufwendig angewendet werden kann.

## Beschreibung

Die Erfindung betrifft ein Pflegesystem zur kosmetischen Behandlung der Haut.

Im Stand der Technik sind die verschiedensten Produkte zur Hautpflege bekannt. Zumeist handelt es sich um Cremes, Ampullen oder unterschiedlich geartete Gesichtsmasken, welche der Haut zumeist Feuchtigkeit spenden und Spannkraft verleihen sollen.

Die im Stand der Technik bekannten Gesichtsmasken bestehen zumeist aus Cremes oder Pasten, die auf die Haut aufgetragen werden und dann pflegend auf diese einwirken. Nachteilig dabei ist, daß sich die Dosierung der Masken oftmals als schwierig erweist. So wird oftmals eine zu große Menge des pflegenden Produktes aufgetragen, wobei der Überschuß im Anschluß an die Behandlung je nach Maske abgewischt oder mit Wasser abgewaschen werden muß. Dies wird zumeist als unangenehm empfunden, weil ein schmieriges Gefühl auf der Haut bleibt und grundsätzlich die Gefahr besteht, daß die Masken in die Haare oder an die Kleidung zu gelangen. Auch müssen für die Entfernung insbesondere von fettigen Produkten oftmals Hilfsmittel wie Tücher oder Watte verwendet werden. Auch erweist sich die Applikation der Masken auf die Haut als umständlich, da die creme- oder pastenartigen Masken mit den Händen aufgetragen werden müssen, wobei bei Gesichtsmasken die Augenpartie und der Mund überwiegend ausgespart werden, da insbesondere die Augenpartie empfindlich ist und in der Regel einer anderen Pflege bedarf als der Rest der Haut. Dies ist umständlich und kann nur unter Zuhilfenahme eines Spiegels erfolgen, da ansonsten die Gefahr besteht, daß die Maske auch auf die empfindliche Augenpartie aufgetragen wird oder nicht gleichmäßig auf die zu behandelnden Partien aufgetragen wird. Neben dieser Unannehmlichkeit verbleiben Reste der Maske an den Händen und diese werden leicht in die Haare, an die Kleidung oder an die Armaturen geschmiert. Daher können die herkömmlichen Masken nicht schnell und unkompliziert zwischendurch angewendet werden, sondern bedürfen einen gewissen Zeitaufwand, da sie nicht schnell und vor allem sauber angewendet werden können. Daher muß der Benutzer für die Pflege seiner Haut einen gewissen Zeitrahmen einplanen, was nicht immer möglich ist und zur Folge hat, daß die Haut oftmals unregelmäßig gepflegt wird.

Ein weiterer Nachteil der im Stand der Technik bekannten Pflegeprodukte besteht darin, daß die Gefahr besteht, daß die Produkte verunreinigt werden können und somit nachteilige Wirkungen insbesondere auf empfindliche Haut haben. So sind Hautreizungen, Ausschläge oder andere Hautirritationen die Folge. Auch bakterielle Infektionen sind möglich. Dies ist darauf zurückzuführen, daß die Pflegeprodukte zumeist in größeren Tuben verkauft werden, die oftmals nicht richtig verschlossen werden. Auch bei ordnungsgemäßer Handhabung verbleiben Reste an der Tubenöffnung, die verunreinigt werden können und bei der nächsten Anwendung auf die Haut gelangen. Des weiteren haben geöffneten Produkte nur eine begrenzte Haltbarkeit, so daß angebrochene Tuben nach einiger Zeit entsorgt werden müssen, was aus Kostengründen unvorteilhaft für den Benutzer ist. Der Benutzer wendet jedoch auch angebrochene Produkte aufgrund eines natürlichen Preisbewußtseins auch oftmals dann noch an, wenn sie eigentlich aus hygienischen Gründen nicht mehr verwendet werden sollten. Auch ist es für den Benutzer oftmals unklar, wie lange ein geöffnetes Produkt angewendet werden darf bzw. wann es geöffnet wurde, was einen erheblichen Unsicherheitsfaktor darstellt. Resultierende Hautreizungen werden dann von dem Benutzer jedoch zumeist mit dem Produkt selbst in Verbindung gebracht, was einen Imageverlust zur Folge haben kann und somit wirtschaftliche Nachteile für den Hersteller verursachen kann.

Es gibt Lösungsansätze, um dieses Problem zu umgehen. So ist es im Stand der Technik beispielsweise bekannt, die Pflegeprodukte zu portionieren, indem die Pflegeprodukte in kleine Ampulleneinheiten abgepackt werden. Zwar umgeht diese Variante das Hygieneproblem, hat aber weiterhin den Nachteil der schwierigen Applikation, da die Aufbringung des Pflegestoffes auf die Haut immer noch mit den Händen erfolgt und bei flüssigen Pflegestoffen immer die Gefahr besteht, daß die Flüssigkeit auf die Kleidung tropft, was neben unerwünschten Flecken ferner den Nachteil hat, Pflegeprodukt zu verschwenden.

Daher liegt vorliegender Erfindung die **Aufgabe** zu Grunde, ein Pflegesystem bereitzustellen, welches den Stand der Technik dahingehend verbessert, daß es einfach und schnell in der Anwendung ist.

**Gelöst** wird diese Aufgabe durch ein Pflegesystem zur kosmetischen Behandlung der Haut durch Auflage eines pflegestoffhaltigen Flächenelements auf die zu behandelnden Hautpartien, umfassend wenigstens ein Textilerzeugnis, welches im wesentlichen flüssigkeitsfrei zusammengepreßt ist, sowie eine Flüssigkeit, wobei das Textilerzeugnis und/oder die Flüssigkeit Pflegestoffe aufweisen und das Textilerzeugnis nach Aufnahme der Flüssigkeit zu einem pflegestoffhaltigen Flächenelement entfaltbar ist.

Das Pflegesystem weist ein Textilerzeugnis auf, das im wesentlichen flüssigkeitsfrei zusammengepreßt ist. Dadurch kann das Textilerzeugnis kompakt verpackt werden und nimmt nur wenig Raum ein und weist zudem nur eine geringe Oberfläche auf. Dadurch wird in vorteilhafter Weise die Handhabung des Textilerzeugnisses vereinfacht und ferner wird eine weitgehende Beschmutzung der Oberfläche verhindert. Im wesentlichen flüssigkeitsfrei bedeutet im Sinne der Erfindung, daß dem flächigen Textilerzeugnis die Feuchtigkeit entzogen wurde und es ferner auf ein geringes Volumen zusammengepreßt wurde. Dadurch entsteht eine trockene, kompakte Einheit mit geringem Volumen.

Ferner weist das Pflegesystem eine Flüssigkeit auf. Beim erfindungsgemäßen Pflegesystem weisen entweder die Flüssigkeit und/oder das Textilerzeugnis die Pflegestoffe auf, wobei die Pflegestoffe vorzugsweise in der Flüssigkeit enthalten sind.

Das Textilerzeugnis ist nach Aufnahme der Flüssigkeit zu einem pflegestoffhaltigen Flächenelement entfaltbar. Um das zusammengepreßte Textilerzeugnis zu einem pflegestoffhaltigem Flächenelement zu entfalten, wird das zusammengepreßte Textilerzeugnis mit der Flüssigkeit des erfindungsgemäßen Systems in Kontakt gebracht. Die Flüssigkeit wird von dem im wesentlichen flüssigkeitsfrei zusammengepreßten Textilerzeugnis aufgenommen. Durch die Flüssigkeitsaufnahme in das Gewebe des Textilerzeugnisses quillt dieses auf und verläßt seine zusammengepreßte Form. Das flüssigkeitsgetränkte Textilerzeugnis ist im aufgequollenen Zustand auffaltungsbereit und kann durch Auffaltung zu einem pflegestoffhaltigen Flächenelement ausgebreitet werden. Das pflegestoffhaltige Flächenelement kann im aufgefalteten Zustand auf die zu behandelnden Hautpartien aufgelegt werden, wo die Pflegestoffe dann direkt auf die Haut einwirken können und diese dadurch gepflegt wird. Dadurch daß die Flüssigkeit samt der Pflegestoffe durch das flächige Textilerzeugnis aufgenommen wird, bildet das Textilerzeugnis im flüssigkeitsgetränkten Zustand eine mit Pflegestoffen getränkte Gewebemaske mit einem pflegenden Feuchtigkeitsfilm. Daß das zusammengepreßte Textilerzeugnis vor der Benutzung im wesentlichen flüssigkeitsfrei ist, hat zudem den Vorteil, daß es primär nur die Flüssigkeit aufnimmt, welche die Pflegestoffe enthält. Die Maske hat dadurch ein größeres Saugvolumen und ist vollständig mit Pflegestoffen getränkt.

Das zu einem pflegestoffhaltigem Flächenelement umgewandelte bzw.aufgefaltete Flächenelement wird auf die zu behandelnde Hautpartie gelegt. Vorzugsweise wird das pflegestoffhaltige Flächenelement leicht auf die Haut angepreßt, um einen guten Kontakt zwischen Textilerzeugnis und Haut zu gewährleisten. Die Pflegestoffe sind im direkten Kontakt mit der Haut und ziehen in diese ein. Dabei ist vorteilhaft, daß bei dem erfindungsgemäßen System im wesentlichen nur so viel Pflegestoffe auf die Haut gelangen, wie diese auch aufnehmen kann, da der Rest in dem flächigen Textilerzeugnis verbleibt und von der Haut nicht aufgenommen wird. Dadurch wird in vorteilhafter Weise umgangen, daß anders als bei herkömmlichen Masken, überschüssige Pflegereste auf der Haut verbleiben, die entfernt werden müssen.

Das flächige Textilerzeugnis verbleibt für einen gewissen Zeitraum auf der Haut und kann nach einer gewissen Einwirkungszeit einfach entsorgt werden. Die Einwirkungsdauer richtet sich im wesentlichen nach dem jeweils in dem Pflegesystem verwendeten Inhalts- bzw. Pflegestoffen, die je nach gewünschtem Effekt variiert werden können. Die Haut kann im Anschluß an die Behandlung direkt weiter behandelt werden, so beispielsweise geschminkt werden, ohne daß Pflegereste erst aufwendig entfernt werden müssen. Dadurch ist das erfindungsgemäße Pflegesystem wenig aufwendig, da auch keine separaten Tücher oder Wattebäuschchen benötigt werden, um überschüssige Reste zu entfernen. Überschüssige Pflegestoffe verbleiben in dem Textilerzeugnis und werden zusammen mit diesem entsorgt. Daher kann das Pflegesystem einfach unterwegs wie beispielsweise auf Reisen, in einem Flugzeug o.ä. angewendet werden.

Das erfindungsgemäße Pflegesystem weist viele Vorteile gegenüber dem Stand der Technik auf. Es ist einfach, sauber und schnell in der Anwendung. Um das Pflegesystem funktionsfähig zu machen, muß lediglich ein zusammengepreßtes Textilerzeugnis mit der Flüssigkeit in Kontakt gebracht werden, wodurch das Textilerzeugnis durch die Flüssigkeitsaufnahme in ein pflegestoffhaltiges Flächenelement umgewandelt werden kann. Dies kann beispielsweise durch Auffaltung des Textilerzeugnisses zu einer pflegenden Textilmaske erfolgen. Es ist in vorteilhafter Weise nicht notwendig, zur Pflege der Haut eine Creme oder eine Paste mit den Fingern im Gesicht zu verteilen. Dadurch wird mit der Erfindung in vorteilhafter Weise umgangen, daß die Hände nach der Pflege gereinigt werden müssen, da für die Benutzung des Pflegesystems nur ein minimaler Kontakt der Pflegestoffe mit den Händen erforderlich ist, da ein Großteil der Pflegestoffe in dem Textilerzeugnis gebunden ist und nur einen Pflegefilm auf dem Flächenelement bilden, so daß die Pflegestoffe im wesentlichen erst bei Kontakt mit der zu behandelnden Hautpartie an diese abgegeben werden. Dabei ist die Menge, anders als bei einer Creme oder Paste, immer verhältnismäßig gering, so daß das Pflegesystem sauber anwendbar ist und die Gefahr von Flecken minimiert wird.

Ferner ist das erfindungsgemäße Pflegesystem leicht zu dosieren. Es wird in vorteilhafter Weise verhindert, daß zuviel oder zuwenig Pflegestoffe auf die Haut gelangen, da diese nur soviel Flüssigkeit aus der getränkten Textilmaske aufnimmt, wie sie benötigt. Das Pflegesystem ist ferner schnell handzuhaben und ist daher auch zwischendurch bequem anwendbar, um sich zu entspannen und die Haut zu erfrischen. Es ist kein Spiegel oder ähnliches notwendig, um das Textilerzeugnis aufzulegen. Das Pflegesystem kann daher auch unterwegs, beispielsweise auf Reisen, gut angewendet werden.

Ein weiterer Vorteil des erfindungsgemäßen Pflegesystems liegt darin, daß es äußerst hygienisch angewendet werden kann. Die für eine Anwendung benötigte Flüssigkeitsmenge wird mit dem zusammengepreßten Textilerzeugnis in Kontakt gebracht und das Textilerzeugnis bildet daher erst durch die Flüssigkeitsaufnahme direkt vor Gebrauch die fertige Maske. Das ist ein Vorteil gegenüber beispielsweise einem System, bei dem die Masken vorgetränkt sind. Es wird verhindert, daß die Maske austrocknet oder beschmutzt wird, da das Textilerzeugnis in seiner zusammengepreßten Form wenig schmutzanfällig ist. Auch wird verhindert, daß sich die Zusammensetzung der Maske durch Lagerung o.ä. verändert, beispielsweise dadurch, daß Wasser verdunstet und sich daher die Konzentration an Pflegestoffen verschiebt. Nach Benutzung der Maske wird diese entsorgt. Dadurch werden Kontaminationen durch Rückstände oder ähnliches verhindert, da keine Wiederverwendung notwendig ist.

Gemäß einer vorteilhaften Weiterbildung wird vorgeschlagen, daß das Textilerzeugnis und die Flüssigkeit im wesentlichen flüssigkeitsdicht verpackt sind. Dadurch wird in vorteilhafter Weise verhindert, daß versehentlich Feuchtigkeit an bzw. in die Komponenten des Pflegesystems gelangt und dieses schädigen, beispielsweise durch ein vorzeitiges auf- bzw. anquellen des komprimierten Textilerzeugnisses oder durch Verdünnung oder Verunreinigung der Flüssigkeit. Dabei kann unter im wesentlichen flüssigkeitsdicht auch eine hygienische bzw. sterile Verpackung subsumiert werden. Letztenendes wird vorgeschlagen, die Verpackung so zu wählen, daß die Gefahr einer Komponentenverunreinigungen und somit von Hautirritationen minimiert wird. Für die Verpackung kommen unterschiedliche Verpackungsmethoden in Betracht, so versiegeln, einschweißen o.a.. Daß das Textilerzeugnis vor dem Kontakt mit der Flüssigkeit des Pflegesystems im wesentlichen flüssigkeitsfrei ist und ferner vorzugsweise flüssigkeitsdicht verpackt ist, hat oben beschriebene Vorteile. Jedoch sind von der Erfindung auch Ausführungsformen umfaßt, bei dem das Textilerzeugnis leicht vorquellt. Dies kann beispielsweise durch unsachgemäße oder zu lange Lagerung geschehen. Eine geringfügige Vorquellung ändert nichts am Grundprinzip des erfindungsgemäßen Pflegesystems, sie ist nur weniger vorteilhaft.

Gemäß einer Weiterbildung wird vorgeschlagen, daß die Flüssigkeit die Haut pflegende Stoffe enthält, und diese Pflegestoffe zusammen mit der Flüssigkeit durch das zusammengepreßte Textilerzeugnis aufnehmbar sind, wobei das Textilerzeugnis nach Aufnahme der Flüssigkeit zu einem pflegestoffhaltigem Flächenelement entfaltbar ist und die Pflegestoffe bei Kontakt des Flächenelements mit der Haut auf diese einwirken. Die Verwendung von Flüssigkeiten mit Pflegestoffen vereinfacht die Herstellung und Handhabung des Pflegesystems und ermöglicht es ferner, eine große Vielzahl von Pflegestoffen anzureichern. Diese können dabei leicht variiert werden. So können beliebige Pflegestoffe bzw. Flüssigkeiten verwendet werden. So ermöglicht auch das Merkmal, daß die Flüssigkeit die Pflegestoffe enthält, daß unterschiedliche Pflegestoffe mit den flächigen Textilerzeugnis variiert werden können. Dadurch ist es möglich, mit einem Textilerzeugnis durch Variation der Inhaltsstoffe der Pflegestoffe unterschiedliche pflegende Masken zu bilden, die jeweils unterschiedliche pflegende Eigenschaften aufweisen. Dadurch können auch unterschiedliche Hautpartien effektiv gepflegt werden.

Gemäß einer vorteilhaften Weiterbildung wird mit der Erfindung vorgeschlagen, daß das flächige Erzeugnis aus einem Naturprodukt, vorzugsweise aus Baumwolle, besteht. Jedoch sind auch Varianten aus Kunststoff möglich und liegen im Rahmen der Erfindung. Vorzugsweise ist das flächige Textilerzeugnis ein Baumwollvlies. Das hat den Vorteil, daß es saugfähig und weich ist, also sich der Haut optimal anpassen kann und als angenehm empfunden wir.

Gemäß einer vorteilhaften Weiterbildung weist das Textilerzeugnis Aussparungen auf. Diese sind von besonders großem Vorteil bei Gesichtsmasken, da dadurch bereits im Textilerzeugnis die Augen- und Mundpartien ausgespart werden können, so daß die Pflegestoffe nicht in diesen Bereichen einwirken. Dadurch wird auf einfache Weise die Notwendigkeit umgangen, daß bei einer Gesichtsbehandlung die Mund- und Augenpartie durch akurates Verteilen der Creme oder Paste umständlich ausgespart werden muß. Dies fördert die leichte Handhabbarkeit des Pflegesystems, da es schnell und unkompliziert benutzt werden kann. Auch wird vorgeschlagen, Einschnitte seitlich der auf der Nase aufliegenden Bereiche vorzunehmen, so daß Nase und Gesicht optimal bedeckt werden und keine Hohlräume unter dem flächigen Textilerzeugnis entstehen.

Dadurch wird eine gute Auflage der Maske und ein enger Hautkontakt ermöglicht. Auch sind andere Formen für die Maske denkbar, je nachdem welche Partie behandelt werden soll. So sind auch Textilerzeugnisse denkbar, die speziell für beispielsweise die Augenpartie oder das Dekolletee geformt sind. Diese können dann mit den entsprechend geeigneten Pflegeprodukten kombiniert werden. Das erfindungsgemäße System kann daher an die verschiedensten Körperpartien angepaßt werden.

Gemäß einer vorteilhaften Weiterbildung wird vorgeschlagen, daß das flächig zusammengepreßte Textilerzeugnis zu einer kompakten Form zusammengepreßt ist, wobei das flächige Textilerzeugnis auf ein minimales Volumen komprimiert ist. Dadurch nimmt das Textilerzeugnis nur wenig Raum ein, was zudem Verpackungsmaterial einspart. Durch die verdichtete Form und die im wesentlichen flüssigkeitsdichte Verpackung wird sichergestellt, daß das flächige Textilerzeugnis im wesentlichen feuchtigkeitsfrei ist und durch die Verpackung auch keine Feuchtigkeit aus der Luft aufnehmen kann. Dadurch wird gewährleistet, daß es bei Kontakt mit der pflegestoffhaltigen Flüssigkeit durch diese gänzlich getränkt wird und nicht bereits ein Teil des Saugpotentials durch nicht pflegestoffhaltige Feuchtigkeit verbraucht wird. Dadurch wird gewährleistet, daß eine hohe Konzentration an pflegenden Stoffen im durchtränkten Textilerzeugnis bzw. im pflegestoffhaltigem Flächenelement vorliegt.. Dabei gibt es unterschiedliche Möglichkeiten, eine derart kompakt komprimierte Form herzustellen, so beispielsweise durch Vakuumpressen o.ä.. Dabei kann die kompakte Form beispielsweise würfelförmig, rechteckig, tabförmig, rund oder mit abgerundeten Kanten sein.

Gemäß einer vorteilhaften Weiterbildung der vorliegenden Erfindung wird ferner vorgeschlagen, daß die Flüssigkeitsmenge, die mit dem kompakt zusammengepreßten flächigen Textilerzeugnis in Kontakt gebracht wird, so gewählt ist, daß das zusammengepreßte Textilerzeugnis durch die Flüssigkeitsaufnahme auf sein volles Volumen ausdehnbar ist und im flüssigkeitsgetränkten Zustand zu einem feuchtem Flächenelement entfaltbar ist. Dadurch wird gewährleistet, daß das Textilerzeugnis vollends einem Flächenelement entfaltet werden kann und dabei gänzlich mit der Flüssigkeit und somit mit Pflegestoffen getränkt ist. Durch einen Feuchtigkeitsüberschuß wird die Übertragung des Feuchtigkeitsfilmes auf die Haut begünstigt. Dies kann durch Anpressung des aufgefalteten Flächenelementes auf die Haut gefördert werden.

Gemäß einer vorteilhaften Weiterbildung wird mit der Erfindung ferner vorgeschlagen, daß Flüssigkeit und flächiges Textilerzeugnis zu einer Funktionseinheit zusammengefaßt sind, wobei diese im wesentlichen aus einem Aufbewahrungsbehältnis mit zwei Kammern besteht, wobei eine Kammer das zusammengepreßte Textilerzeugnis aufweist und die andere eine pflegestoffhaltige Flüssigkeit, wobei das Tab von der einen Kammer in die die Flüssigkeit enthaltende Kammer überführbar ist und das Textilerzeugnis nach Aufnahme der Flüssigkeit zu einem pflegestoffhaltigen Flächenelement entfaltbar ist. Dadurch wird das Pflegesystem in vorteilhafter Weise für den hygienischen Einmalgebrauch bereitgestellt. Durch diese Vorrichtung wird in vorteilhafter Weise eine schnell nutzbare Einheit zur Verfügung gestellt, die nach dem Gebrauch sofort entsorgt werden kann. Die Zusammenfassung des Pflegesystems in eine derartige Vorrichtung ist insbesondere vorteilhaft für den Gebrauch unterwegs, so beispielsweise auf Reisen. Die Vorrichtung kann flüssigkeitsdicht versiegelt werden, so daß verhindert wird, daß in die Kammern Feuchtigkeit eindringen kann. Dadurch wird der Zustand von Textilerzeugnis und Flüssigkeit bis zum Gebrauch konserviert. Dabei ist vorteilhaft, wenn das Flüssigkeitsreservoir genau die Menge an Flüssigkeit enthält, die benötigt wird, um das zusammengepreßte Textilerzeugnis vollständig aufzuquellen und auffaltungsbereit zu machen.

Ferner soll mit der Erfindung ein Verfahren zur kosmetischen Behandlung der Haut durch Auflage eines pflegestoffhaltigen Flächenelements unter Schutz gestellt werden, welches dadurch gekennzeichnet ist, daß ein im wesentlichen flüssigkeitsfrei zu einer kompakten Form zusammengepreßtes flächiges Textilerzeugnis mit einer Flüssigkeit in Kontakt gebracht wird, wobei die Flüssigkeit und/oder das flächige Textilerzeugnis die Haut pflegende Stoffe aufweisen, das Textilerzeugnis die Flüssigkeit aufnimmt und das Textilerzeugnis im flüssigkeitsgetränkten Zustand zu einem pflegestoffhaltigem Flächenelement entfaltet wird und auf die zu behandelnden Partien der Haut aufgelegt wird. Dieses Verfahren kann mit einem Pflegesystem nach den Ansprüchen 1 bis 9 durchgeführt werden.

Weitere Vorteile und Merkmale ergeben sich anhand der Figuren und der dazugehörigen Beschreibung. Diese dienen ausschließlich der näheren Erläuterung und zeigen einige mögliche Ausgestaltungen des erfindungsgemäßen Pflegesystems. Dabei zeigen:
- Figur 1a bis 1 d: das erfindungsgemäße Pflegesystem gemäß einer Ausführungsvariante, sowie die Benutzung dieses,
- Figur 2a bis 2d: das erfindungsgemäße Pflegesystem gemäß einer weiteren Ausführungsvariante, sowie die Anwendung dieses Pflegesystems.

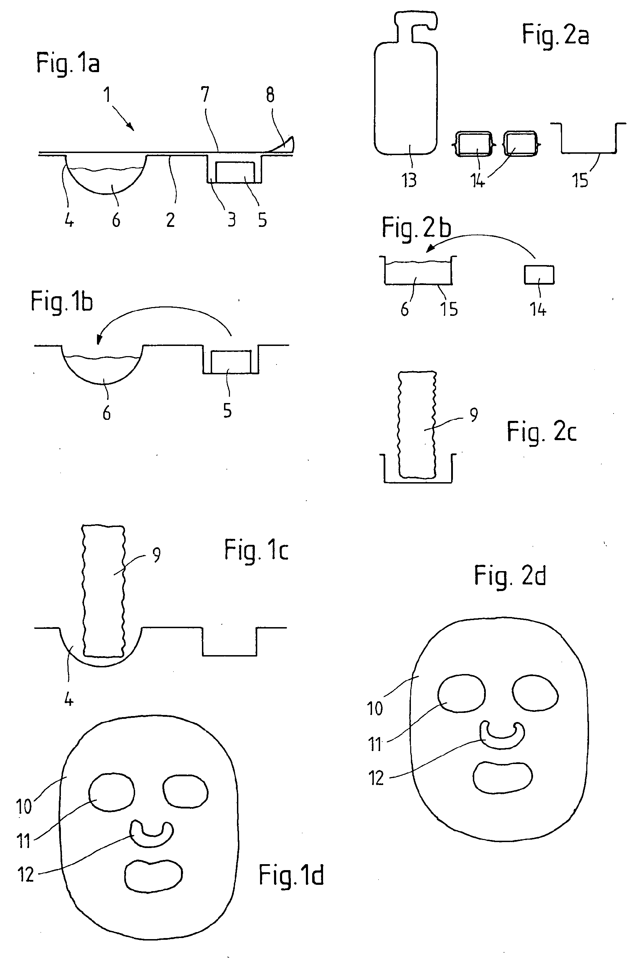

In Figur 1a ist ein erfindungsgemäßes Pflegesystem im versiegelten Zustand dargestellt. Dabei ist es gemäß Ausführungsbeispiel zu einer Funktionseinheit 1 zusammengefaßt. Diese besteht aus einem Aufbewahrungsbehältnis 2 aus Kunststoff, das zwei Kammern 3 und 4 aufweist. In der etwas kleiner gestalteten Kammer 3 befindet sich das zu einem kompakten Tab komprimierte Textilerzeugnis 5. In der Kammer 4 befindet sich die Flüssigkeit 6, welche die Haut pflegende Stoffe aufweist. Um sowohl das kompakt zusammengepreßte Textilerzeugnis 5 als auch die Flüssigkeit 6 vor Verunreinigungen und vor Feuchtigkeit zu schützen, ist das Aufbewahrungsbehältnis mit einem Deckel 7 versiegelt. Mittels der Aufreißlasche 8 kann der Deckel 7 einfach von dem Aufbewahrungsbehältnis 2 entfernt werden, so daß die beiden Kammern 3 und 4 freigelegt werden. Das zu einem kompakten Tab komprimierte Textilerzeugnis 5 wird aus der Kammer 3 genommen und in die mit Flüssigkeit gefüllte Kammer 4 überführt (Figur 1b). Durch den Kontakt mit der Flüssigkeit 6 quillt das Tab 5 auf und das Textilerzeugnis dehnt sich auf seine Normalgröße 9 durch die Flüssigkeitsaufnahme auf. Dabei ist es vorteilhaft, wenn das Reservoir 4 gerade mit so viel Flüssigkeit gefüllt ist, daß sie ausreicht, um das komprimierte Textilerzeugnis 5 in ein pflegestoffhaltiges Flächenelement 9, welches auffaltungsbereit ist, umzuwandeln (Figur 1c). Bei einer Maske aus Baumwollvlies, die beispielsweise zur Auflage auf die Gesichtspartie geeignet ist, sind ca. 6ml ausreichend. Dies ist aber abhängig von der Größe der Maske sowie vom verwendeten Textilerzeugnismaterial und kann daher variieren. Durch die genaue Anpassung der Flüssigkeitsmenge an das Saugpotential des eingesetzten Textilerzeugnisses wird in vorteilhafter Weise verhindert, daß übermäßig viel Feuchtigkeitsreste in der Kammer 4 zurückbleiben, was neben einer Verschwendung von Pflegeprodukt auch unsauberer wäre, da diese Reste auslaufen könnten. Figur 1d zeigt das pflegestoffhaltige Flächenelement 10 in ausgefalteter Form. Die Auffaltung kann einfacher Weise mit den Daumen und Zeigefingern erfolgen. Dadurch verbleiben Pflegereste nur an den Fingerspitzen von je zwei Fingern, wo sie schnell einziehen. Im aufgefalteten Zustand kann das pflegestoffhaltige Flächenelement auf die zu behandelnde Hautpartie aufgelegt werden. Im vorliegenden Ausführungsbeispiel handelt es sich bei dem Textilerzeugnis um ein Baumwollvlies, welches in vorteilhafter Weise Aussparungen 11 aufweist. Diese Aussparungen 11 in der Maske 10 sparen die Mund und Augenpartie des Gesichts aus und bewirken in vorteilhafter Weise ferner aufgrund der Nasenschlitze 12, daß das Textilerzeugnis 10 sich optimal an die Gesichtskonturen anpassen kann. Durch die Aussparungen 11 kann der Benutzter die Maske einfach aufs Gesicht legen und leicht auf die Haut anpressen, um einen optimalen Kontakt zwischen der Haut und der mit Pflegestoffen getränkten Textilmaske 10 herzustellen. Durch die Aussparungen 11 ist es nicht notwendig, bei der Anwendung der Maske besondere Vorsicht walten zu lassen, um einen Kontakt zwischen den Pflegeprodukten und den empfindlichen Augen- und Lippenpartien zu verhindern. Dadurch ist die Maske 10 einfach in der Anwendung und kann auch schnell zwischendurch gebraucht werden, ohne daß besondere Vorkehrungen getroffen werden müssen.

Figur 2a bis 2d zeigt wie Figur 1a bis 1d das erfindungsgemäße Pflegesystem und seine Anwendung gemäß einer weiteren Ausführungsvariante. Gemäß dieser Ausführungsvariante befindet sich die pflegende Flüssigkeit 6 in einem Flüssigkeitsspender 13 und die Textiltabs 14 sind einzeln verpackt. Das Pflegesystem ist in der Form beispielsweise für Mehrpersonenhaushalte geeignet, oder wenn die Pflege häufig erfolgen soll. Durch die einzelne Verpackung sind die kompakt zusammengepreßten Textilerzeugnisse 14 vor Feuchtigkeit geschützt und können beispielsweise in einer Box zusammen mit dem Flüssigkeitsspender verkauft werden. Auch ist möglich, Flüssigkeiten mit unterschiedlichen Pflegestoffen zu verkaufen, welche dann mit den Textilerzeugnissen 14 kombiniert werden können. So ist es möglich, mit dem Pflegesystem unterschiedlich wirkende Masken herzustellen, sofern Flüssigkeiten mit unterschiedlichen Pflegestoffen zur Verfügung stehen. Die die Pflegestoffe enthaltende Flüssigkeit wird in ein Behältnis 15 gegeben. Dabei kann dieses Beispielsweise Randmarkierungen aufweisen, um die für die völlige Entfaltung des Textilerzeugnisses notwendige bzw. ausreichende Menge an Flüssigkeit anzugeben. Auch ist vorstellbar, daß der Pumpspender so eingestellt wird, daß bei einmaliger Betätigung die erforderliche Menge an Flüssigkeit dosiert abgegeben wird. Das kompakt komprimierte Textilerzeugnis 14 wird in den Behälter 15 mit der die Pflegestoffe enthaltenden Flüssigkeit 6 überführt (Figur 2b). Durch die Feuchtigkeit quillt das Textilerzeugnis auf und nimmt die Flüssigkeit samt der Pflegestoffe in sein Gewebe auf und ist dann in der flüssigkeitsgetränkten, expandierten Form 9 (Figur 2c). Das mit Pflegestoffen durchtränkte und expandierte Textilerzeugnis kann zu einem pflegestoffhaltigem Flächenelement 10 entfaltet werden und zur Behandlung auf die zu pflegenden Hautpartien aufgelegt werden. Nach Benutzung kann dieses einfach und sauber entsorgt werden, ohne die Gefahr, daß Reste verschmiert werden.

Auch besteht die Möglichkeit, daß das Textilerzeugnis die Pflegestoffe enthält. Dies ist beispielsweise dann vorteilhaft, wenn die Pflegestoffe durch den Preßvorgang nicht geschädigt werden. Durch Kontakt des pflegestoffhaltigen zusammengepreßten Textilerzeugnisses mit einer Flüssigkeit wie beispielsweise Wasser quillt das Textilerzeugnis auf und die Pflegestoffe werden gelöst. Dadurch wird eine pflegestoffhaltige Maske gebildet, die durch Auffaltung zu einem Flächenelement ausgebreitet werden kann, welches dann auf die zu behandelnden Hautpartien aufgelegt werden kann.

### Bezugszeichenliste

- 1: Funktionseinheit
- 2: Aufbewahrungsbehältnis
- 3: Kammer für Textilerzeugnis
- 4: Kammer für Flüssigkeit
- 5: komprimiertes Textilerzeugnis
- 6: Flüssigkeit mit Pflegestoffen
- 7: Deckel
- 8: Aufreißlasche
- 9: pflegestoffhaltiges Flächenelement
- 10: entfaltetes pflegestoffhaltiges Flächenelement
- 11: Aussparung
- 12: Nasenschlitze
- 13: Flüssigkeitsspender
- 14: komprimiertes Textilerzeugnis
- 15: Behältnis

## Patentansprüche

1. Pflegesystem zur kosmetischen Behandlung der Haut durch Auflage eines pflegestoffhaltigen Flächenelements auf die zu behandelnden Hautpartien, umfassend wenigstens ein Textilerzeugnis, welches im wesentlichen flüssigkeitsfrei zusammengepreßt ist, sowie eine Flüssigkeit, wobei das Textilerzeugnis und/oder die Flüssigkeit Pflegestoffe aufweisen und das Textilerzeugnis nach Aufnahme der Flüssigkeit zu einem pflegestoffhaltigen Flächenelement entfaltbar ist.

2. Pflegesystem nach Anspruch 1, **dadurch gekennzeichnet, daß** das Textilerzeugnis und die Flüssigkeit im wesentlichen flüssigkeitsdicht verpackt sind.

3. Pflegesystem nach Anspruch 1 und 2, **dadurch gekennzeichnet, daß** die Flüssigkeit die Haut pflegende Stoffe enthält und diese Pflegestoffe zusammen mit der Flüssigkeit durch das zusammengepreßte Textilerzeugnis aufnehmbar sind, wobei das Textilerzeugnis im flüssigkeitsgetränkten Zustand zu einem pflegestoffhaltigen Flächenelement entfaltbar ist und die Pflegestoffe bei Kontakt des Flächenelements mit der Haut auf diese einwirken.

4. Pflegesystem nach einem oder mehreren der Ansprüche 1 bis 3, **dadurch gekennzeichnet, daß** das Textilerzeugnis aus Baumwolle ist.

5. Pflegesystem nach einem oder mehreren der Ansprüche 1 bis 4, **dadurch gekennzeichnet, daß** das Textilerzeugnis Aussparungen aufweist.

6. Pflegesystem nach einem oder mehreren der Ansprüche 1 bis 5, **dadurch gekennzeichnet, daß** das Textilerzeugnis zu einer kompakten Form zusammengepreßt ist, wobei das Textilerzeugnis auf ein minimales Volumen komprimiert ist.

7. Pflegesystem nach einem oder mehreren der Ansprüche 1 bis 6, **dadurch gekennzeichnet, daß** die Flüssigkeitsmenge, die mit dem zusammengepreßten Textilerzeugnis in Kontakt gebracht wird so gewählt ist, daß das zusammengepreßte Textilerzeugnis durch die Flüssigkeitsaufnahme auf sein volles Volumen ausdehnbar ist und im flüssigkeitsgetränkten Zustand zu einem feuchten Flächenelement entfaltbar ist.

8. Pflegesystem nach einem oder mehreren der Ansprüche 1 bis 7, **dadurch gekennzeichnet, daß** Flüssigkeit und flächiges Textilerzeugnis zu einer Funktionseinheit zusammengefaßt sind, wobei diese im wesentlichen aus einem Aufbewahrungsbehältnis mit zwei Kammern besteht, wobei eine Kammer das zusammengepreßte Textilerzeugnis aufweist und die andere eine pflegestoffhaltige Flüssigkeit, wobei das zusammengepreßte Textilerzeugnis in die die Flüssigkeit enthaltende Kammer überführbar ist und das Textilerzeugnis nach Aufnahme der Flüssigkeit zu einem pflegestoffhaltigen Flächenelement entfaltbar ist.

9. Pflegesystem nach Anspruch 8, **dadurch gekennzeichnet, daß** die Vorrichtung flüssigkeitsdicht versiegelt ist.

10. Verfahren zur kosmetischen Behandlung der Haut durch Auflage eines pflegestoffhaltigen Flächenelements, **dadurch gekennzeichnet,**
- **daß** ein im wesentlichen flüssigkeitsfrei zusammengepreßtes Textilerzeugnis mit einer Flüssigkeit in Kontakt gebracht wird, wobei die Flüssigkeit und/oder das flächige Textilerzeugnis die Haut pflegende Stoffe aufweisen,
- das Textilerzeugnis die Flüssigkeit aufnimmt,
- das Textilerzeugnis im flüssigkeitsgetränkten Zustand zu einem pflegestoffhaltigen Flächenelement entfaltet wird und das Flächenelement auf die zu behandelnden Partien der Haut aufgelegt wird.
